# EUROPEAN PATENT SPECIFICATION

(11) **EP 2 676 694 B1**
(45) Date of publication and mention of the grant of the patent: **26.04.2017**
(21) Application number: 13004526.3
(22) Date of filing: 12.06.2008
(51) Int. Cl.: A61M 15/00

(54) **Inhaler**
Inhalator
Inhalateur

(30) Priority: 15.06.2007 EP 07011750; 11.12.2007 EP 07023920
(43) Date of publication of application: 25.12.2013
(62) Divisional of application: 08773393.7
(73) Proprietor: Boehringer Ingelheim International GmbH, 55216 Ingelheim am Rhein (DE)
(72) Inventor: Wachtel, Herbert, 55216 Ingelheim am Rhein (DE); Moser, Achim, 55216 Ingelheim am Rhein (DE)
(74) Representative: Von Rohr Patentanwälte Partnerschaft mbB

(56) References cited:
- WO-A-00/21594
- WO-A-03/084502
- WO-A1-95/28980
- US-A- 5 672 581
- US-A- 6 116 237
- US-A1- 2005 183 718

## Description

The present invention relates to an inhaler according to the preamble of claim 1.

The present invention specifically relates to active inhalers such as an inhaler sold under the brand name "Respimat" in the form of an inhaler, as illustrated in its basic structure in WO 91/14468 A1 and in a specific embodiment in WO 97/12687 A1 (Figs. 6a, 6b). The inhaler has, as a reservoir for a fluid, which is to be atomised, an insertable rigid container having an inner bag containing the fluid and a pressure generator with a drive spring for delivering and atomising the fluid.

The invention also relates to powder inhalers, whether multidose pre-metered or reservoir devices.

To supplement the disclosure of the present application reference is made to the complete disclosure of both WO 91/14468 A1 and WO 97/12687 A1. Generally, the disclosure contained therein preferably relates to a inhaler with a spring pressure of 5 to 200 MPa, preferably 10 to 100 MPa on the fluid, with a volume of fluid delivered per stroke of 10 to 50 µl, preferably 10 to 20 µl, most preferably about 15 µl. The fluid is converted into an aerosol the droplets of which have an aerodynamic diameter of up to 20 µm, preferably 3 to 10 µm. Furthermore, the disclosure contained therein preferably relates to a inhaler of cylindrical shape about 9 cm to about 15 cm and about 2 cm to about 5 cm wide and with a jet spray angle of 20° to 160°, preferably 80° to 100°. These values also apply to the inhaler according to the teaching of the present invention as particularly preferred values.

Active inhalers in the sense of the present invention generate the desired aerosol of the inhalation formulation by means of a conveying means, such as a propellant, a pump, an air pump or any other pressure generator or compressed or liquefied gas, i.e. not due to an air stream of a patient or user who breathes in although the dispensing operation (aerosol operation) can be triggered by the breathing in.

When a patient puts a mouthpiece or any other end piece in his mouth and breathes in, an air stream of ambient air is sucked through the inhaler to entrain the already generated aerosol of the inhalation formulation and to discharge this aerosol. A pressure drop occurs within the inhaler when the air stream flows through the inhaler. This pressure drop depends on the flow rate and flow velocity. The flow resistance represents a quantity relating to the square root of the pressure drop at a certain flow rate.

In the present invention, the term "flow resistance" means the resistance, which occurs when air is sucked from the mouthpiece or any other end piece of the inhaler during inhalation. In particular, the flow resistance relates to a flow path for ambient air through at least one air supply opening of the inhaler into a mouthpiece of the inhaler. More preferably, the flow resistance means the total flow resistance of the inhaler in the present invention, even if the inhaler has multiple air supply openings through which air can be sucked into the mouthpiece.

In contrast to active inhalers, where an active means generates the aerosol, passive inhalers usually have a higher flow resistance for the patient or user. This results from the fact that the generation of aerosol requires energy and, thus, leads to a respective flow resistance in passive inhalers. Nevertheless, it has been an object of the previous developments to keep the flow resistance as low as possible in both types of inhalers in order to facilitate inhalation for the patient or user.

US 2005/0183718 A1 discloses an axially moveable valve element arranged in a mouthpiece.

US 5,724,960 discloses an MDI with an insert that is fixed on a support part, namely inserted into an expulsion duct upstream of a nozzle and a mouthpiece.

WO 2006/094796 A1 discloses an inhaler with a mixing channel for producing an aerosol. A perforated plate can be placed at or before the inlet opening of the mixing channel in order to increase the flow resistance.

Object of the present invention is to provide an active inhaler with optimized discharge characteristics.

The above object is achieved by an inhaler according to claim 1. Advantageous embodiments are subject of the subclaims.

According to an aspect of the present invention, the inhaler comprises an insert in the mouthpiece in order to define or increase the flow resistance. This allows optimized discharge characteristics. The insert comprises at least one sealing lip to avoid any undesired bypass flow and to achieve a desired clamping within the mouthpiece.

Preferably, the flow resistance of the inhaler is increased. In particular, the inhaler has a flow resistance of at least 60000 Pa^{1/2}s/m³. This is significantly higher than previous inhalers of the present type. Such inhalers usually have a flow resistance of only about 16000 Pa^{1/2}s/m³.

The increased flow resistance results in optimized discharged characteristics of the inhaler. Due to the higher flow resistance the patient or user will breathe in more slowly so that the total inhalation time is increased. In particular, the higher flow resistance makes it easier for the patient or user to slowly breathe in. This applies to intuitive breathing as well as to willful breathing. Thus, a lower flow velocity can be achieved in the mouth and pharynx region, which results in lower impaction of the aerosol particles (inhalation formulation) in this region so that the percentage of lung deposition of the inhalation formulation is increased.

Further aspects, features, properties and advantages of the present invention are described in or the claims and the subsequent description of a preferred embodiment, with reference to the drawing. There are shown in:
- Fig. 1: shows a schematic section of an inhaler in the non-tensioned state;
- Fig. 2: shows a schematic section, rotated by 90° compared with Fig. 1, of the inhaler in the tensioned state;
- Fig. 3a: shows schematically an insert, which can be mounted or inserted in a mouthpiece of the inhaler;
- Fig. 3b: shows a schematic side view of the insert according to Fig. 3a;
- Fig. 3c: shows another schematic side view of the insert perpendicular to the view according to Fig. 3b;
- Fig. 3d: shows a schematic section of the insert according to line IIIb-IIIb of Fig. 3a; and
- Fig. 4: shows a schematic section of the inhaler with the insert.

In the Figures, the same reference numbers are used for identical or similar parts, even if a repeated description is omitted. In particular identical or corresponding advantages and properties then also result or may be achieved.

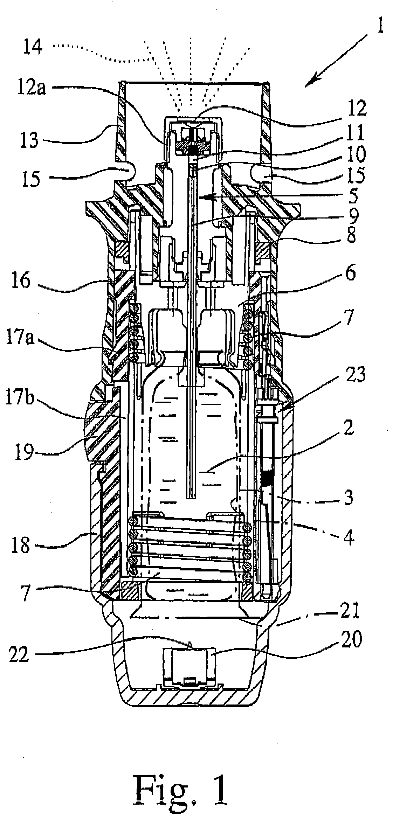

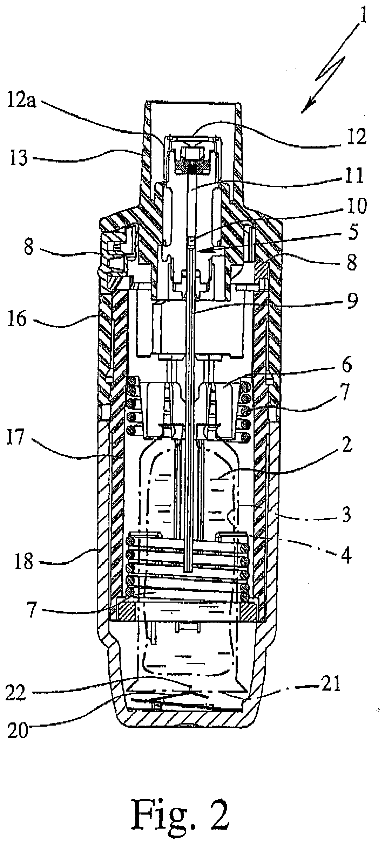

Figs. 1 and 2 show an inhaler 1 according to the present invention for atomising an inhalation formulation 2 as an aerosol, particularly a highly effective pharmaceutical composition or the like, diagrammatically shown in the non-tensioned state (Fig. 1) and in the tensioned state (Fig. 2).

The term "aerosol" in this respect is not limited to an inhalation formulation in liquid from, but also encompasses powder formulations.

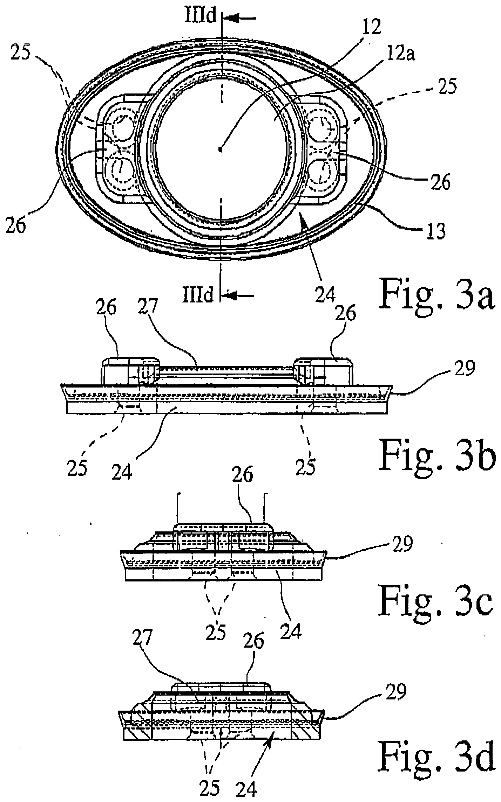

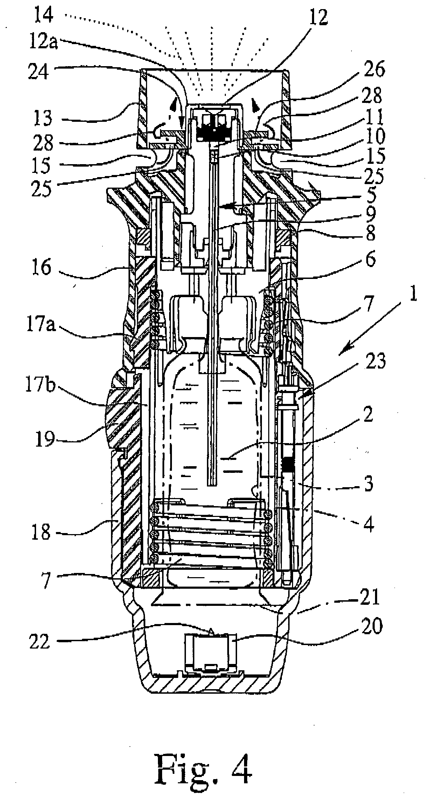

Fig. 3 and 4 show an insert 24 for an inhaler 1, whereas the insert 24 can be inserted in the mouthpiece 13 of the inhaler.

The inhaler 1 is constructed in particular as a portable inhaler and preferably operates without propellant gas. However, the present invention may also be applied to inhalers 1 using a propellant, such as so-called MDIs (metered dose inhalers), a gas, such as compressed or liquefied gas or air, or the like, i.e. in particular to all kind of inhalers 1.

The inhalation formulation 2 is preferably a liquid, in particular a solution, suspension or suslution (mixture of solution and suspension), but can have any form and can be e.g. a powder or the like.

When the inhalation formulation 2, preferably a liquid, more particularly a pharmaceutical composition, is nebulised, an aerosol is formed, which can be breathed in or inhaled by a user (not shown). Usually the inhaling is done at least once a day, more particularly several times a day, preferably at set intervals, depending on the complain from which the patient is suffering.

The inhaler 1 has in particular an insertable and preferably exchangeable container 3 containing the inhalation formulation 2. The container thus forms a reservoir for the inhalation formulation 2, which is to be nebulised. Preferably, the container 3 contains an amount of inhalation formulation 2 or active substance which is sufficient to provide up to 200 dosage units, for example, i.e. to allow up to 200 sprays or applications. A typical container 3, as disclosed in WO 96/06011 A1, holds a volume of about 2 to 10 ml.

The container 3 is substantially cylindrical or cartridge-shaped and once the inhaler 1 has been opened the container can be inserted therein from below and changed if desired. It is preferably of rigid construction, the inhalation formulation 2 in particular being held in a collapsible bag 4 in the container 3.

The inhaler 1 has a conveying means, such as a propellant, a pump, an air pump or any other pressure generator or compressed or liquefied gas, in particular a pump or pressure generator 5 for conveying gas, any other fluid and/or the inhalation formulation 2 and for nebulising the inhalation formulation 2, particularly in a preset and optionally adjustable dosage amount.

The inhalation formulation 2 may be metered in the inhaler 1 as it is the case in the present embodiment or may be pre-metered in an appropriate storage means, such as a blister with multiple blister pockets or the like.

In the present embodiment, the pressure generator 5 has preferably a holder 6 for the container 3, an associated drive spring 7, only partly shown, with a locking element 8 which can be manually operated to release it, a conveying tube 9 with a non-return valve 10, a pressure chamber 11 and/or an expulsion nozzle 12 in the region of a mouthpiece 13. The container 3 is fixed in the inhaler 1 via the holder 6 such that the conveying tube 9 penetrates into the container 3. The holder 6 may be constructed so that the container 3 is able to be exchanged.

As the drive spring 7 is axially tensioned the holder 6 with the container 3 and the conveying tube 9 is moved downwards in the drawings, and the inhalation formulation 2 is sucked out of the container 3 into the pressure chamber 11 of the pressure generator 5 through the non-retum valve 10.

During the subsequent relaxation after actuation of the locking element 8 the inhalation formulation 2 in the pressure chamber 11 is put under pressure as the conveying tube 9 with its now closed non-return valve 10 is moved back upwards by the relaxation of the drive spring 7 and now acts as a pressing ram or piston. This pressure forces the inhalation formulation 2 through the expulsion or dispensing nozzle 12, whereupon it is nebulised into an aerosol 14, as shown in Fig. 1. The droplet size of the particles for a device of the Respimat type has already been discussed hereinbefore.

A user (not shown) can inhale the aerosol 14, while an air supply is sucked into the mouthpiece 13 through at least one air supply opening 15, preferably multiple air supply openings 15. Thus, a bypass is formed so that ambient air can be sucked into the mouthpiece 13.

The inhaler 1 comprises an upper housing part 16 and an inner part 17 which is rotatable relative thereto (Fig. 2) having an upper part 17a and a lower part 17b (Fig. 1), while an in particular manually operable housing part 18 is releasably fixed, particularly fitted onto the inner part 17, preferably by means of a retaining element 19. In order to insert and/or replace the container 3 the housing part 18 can be detached from the inhaler 1.

The inhaler 1 further comprises one or more air supply openings 15 with a reduced size compared to the state of the art. Alternatively or additionally, an insert 14 can be inserted in the mouthpiece 13 of the inhaler 1 as explained later.

The housing part 18 can be rotated relative to the upper housing part 16, carrying with it the part 17b of the inner part 17, which is lower down in the drawings. As a result the drive spring 7 is tensioned in the axial direction by means of a gear (not shown) acting on the holder 6. During tensioning the container 3 is moved axially downwards until the container 3 assumes an end position as shown in Fig. 2. In this state the drive spring 7 is under tension. During the nebulising process the container 3 is moved back into its original position by the drive spring 7. Thus the container 3 executes a lifting movement during the tensioning process and during the atomising process.

The housing part 18 preferably forms a cap-like lower housing part and fits around or over a lower free end portion of the container 3. As the drive spring 7 is tensioned the container 3 moves with its end portion (further) into the housing part 18 or towards the end face thereof, while an axially acting spring 20 arranged in the housing part 18 comes to bear on the base 21 of the container and pierces the container 3 or a base seal thereon with a piercing element 22 when the container makes contact with it for the first time, to allow air in.

The inhaler 1 comprises a monitoring device 23, which counts the actuations of the inhaler 1, preferably by detecting the rotation of the inner part 17 relative to the upper part 16 of the housing.

The total size of the air supply opening(s) 15 (at least essentially) defines or sets the flow resistance of the inhaler 1 when a user (not shown) puts the mouthpiece 13 in his mouth and breathes in to inhale the aerosol 14. This size is significantly reduced according to the present invention. Therefore, the inhaler 1 has a flow resistance of at least 60000 Pa^{1/2}s/m³, in particular of at least 75000 Pa^{1/2}s/m³, more preferably about 90000 or 96000 Pa^{1/2}s/m³ or more.

The flow resistance is measured here as the quotient of the square root of the pressure drop divided by the flow rate. For example, a pressure drop of 4000 Pa results at a flow rate of 39 I/min in a flow resistance of 97301 Pa^{1/2}s/m³.

Thus, a relatively slow inhalation can be achieved. In particular, the duration of the inhalation can be prolonged and/or a relatively low flow velocity or rate through the mouthpiece 8 or its outlet tube 18 can be achieved, even if the patient or user breathes in only intuitively.

In order to define or increase the flow resistance as mentioned above, the at least one air supply opening 15 or multiple air supply openings 15 may be reduced in cross section. In particular, the following diameters may be chosen, wherein the diameter relate to a circular aerodynamic cross section corresponding in its effect to the actual cross section of the respective opening 15: diameter 3.6 to 3.2 mm if the inhaler comprises only one opening 15; diameter 2.6 to 2.2 mm if the inhaler 1 comprises two openings 15; diameter 2.1 to 1.8 mm if the inhaler 1 comprises three openings 15; diameter 1.8 to 1.5 mm if the inhaler 1 comprises four openings 15. Thus, a (total) flow resistance of at least about 60.000 pa^{1/2}s/m³ to 96.000 pa^{1/2}s/m³ can be achieved.

As already mentioned, an insert 24 may be used to define or increase the flow resistance as mentioned above, and/or to create a device flow path 28 or flow characteristics as explained later. Fig. 3a shows a view into the open mouthpiece 13 of the inhaler 1 with the insert 24. Figs. 3b to 3d show different views of the insert 24. Fig. 4 shows a schematic section of the inhaler 1 with the insert 24.

The insert 24 is inserted into the mouthpiece 13, preferably sealingly between the inner wall of the mouthpiece 13 and a nozzle protrusion 12a. The nozzle protrusion 12a preferably protrudes into the mouthpiece 13 and/or is preferably at least essentially cylindrical. The nozzle protrusion 12a preferably holds or forms the nozzle 12. However, other constructional solutions are possible as well.

The shown insert 24 is essentially flat or plate-like. However, other constructional solutions are possible as well.

The insert 24 preferably comprises a central recess 27 for the nozzle protrusion 12a in order to allow the preferred mounting between the outer or peripheral surface of the nozzle protrusion 12a and the inner surface of the mouthpiece 13.

Preferably, the insert 24 is clamped into the mouthpiece 13 as described. However, it can be fastened in any suitable manner.

The insert 24 comprises at least one parallel sealing lip 29 to avoid any undesired bypass flow and to achieve a desired clamping within the mouthpiece 13. However, the insert 24 can also be press-fitted into the mouthpiece 13 and/or onto the nozzle protrusion 12a.

The insert 24 comprises preferably at least one hole 25, in the shown embodiment four holes 25, through which ambient air, which has been sucked in through the openings 15, can flow.

The air stream 28 of bypass air flowing through the at least one hole 25 or any other opening is preferably deflected or guided preferably by the insert 24 in order slow down the flow velocity, and/or to generate turbulences, and/or to allow optimized guidance of the aerosol 14 into the mouth of a user (not shown), and/or to allow optimized mixing of the bypass or ambient air with the aerosol 14.

In the shown embodiment, the insert 24 comprises at least one guiding element 26, here two guiding elements 26, which deflect(s) the air flow (schematically shown in Fig. 4) through the associated hole(s) 25 in the desired manner. In particular, the guiding element 26 blocks a direct or straight flow of the bypass or ambient air through the mouthpiece 13. In the present embodiment, the guiding element 26 is spaced to its associated hole(s) 25 and/or covers the associated hole(s) 25 and/or extends essentially transversally to the main flow direction or outlet direction of the inhaler 1.

The at least one hole 25 may be used to define or increase the (total) flow resistance of the inhaler 1 as mentioned above and/or to create a device flow path 28 or desired flow characteristics. In particular the at least one hole 25 may comprise an aerodynamic cross section with a diameter depending on the number of the holes 25 and dimensioned as mentioned above for the openings 15, i.e. in case of four holes 25 each hole 25 comprises preferably an aerodynamic cross section with a diameter of about 1.8 to 1.5 mm.

In the shown embodiment, two guiding elements 26 are provided each covering or associated to two holes 25.

However, other constructional solutions are possible as well.

The insert 24 may be mounted optionally. Additionally or alternatively, different inserts 24 can be mounted depending on the respective inhalation formulation 2 to achieve the desired results. In particular, to adapt the flow resistance and/or characteristic of the bypass air to the respective inhalation formulation 2.

Preferably, the inhaler 1 is portable, works only mechanically and/or is handheld.

Some preferred ingredients and/or compositions of the preferably medicinal formulation 2 are listed below. As already mentioned, they are in particular powders or liquids in the broadest sense. Particularly preferably the formulation 2 contains the following:
The compounds listed below may be used in the device according to the invention on their own or in combination. In the compounds mentioned below, **W** is a pharmacologically active substance and is selected (for example) from among the betamimetics, anticholinergics, corticosteroids, PDE4-inhibitors, LTD4-antagonists, EGFR-inhibitors, dopamine agonists, H1-antihistamines, PAF-antagonists and PI3-kinase inhibitors. Moreover, double or triple combinations of **W** may be combined and used in the device according to the invention. Combinations of **W** might be, for example:
   - **W** denotes a betamimetic, combined with an anticholinergic, corticosteroid, PDE4-inhibitor, EGFR-inhibitor or LTD4-antagonist,
   - **W** denotes an anticholinergic, combined with a betamimetic, corticosteroid, PDE4-inhibitor, EGFR-inhibitor or LTD4-antagonist,
   - **W** denotes a corticosteroid, combined with a PDE4-inhibitor, EGFR-inhibitor or LTD4-antagonist
   - **W** denotes a PDE4-inhibitor, combined with an EGFR-inhibitor or LTD4-antagonist
   - **W** denotes an EGFR-inhibitor, combined with an LTD4-antagonist.

The compounds used as betamimetics are preferably compounds selected from among albuterol, arformoterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orci-prenaline, pirbuterol, procaterol, reproterol, rimiterol, ritodrine, salmefamol, salmeterol, soterenol, sulphonterol, terbutaline, tiaramide, tolubuterol, zinterol, CHF-1035, HOKU-81, KUL-1248 and
- 3-(4-{6-[2-hydroxy-2-(4-hydroxy-3-hydroxymethyl-phenyl)-ethylamino]-hexyloxy}-butyl)-benzyl-sulphonamide
- 5-[2-(5.6-diethyl-indan-2-ylamino)-1-hydroxy-ethyl]-8-hydroxy-1H-quinolin-2-one
- 4-hydroxy-7-[2-{[2-{[3-(2-phenylethoxy)propyl]sulphonyl}ethyl]-amino}ethyl]-2(3H)-benzothiazolone
- 1-(2-fluoro-4-hydroxyphenyl)-2-[4-(1-benzimidazolyl)-2-methyl-2-butylamino]ethanol
- 1-[3-(4-methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminaphenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-hydroxy-8-(1-hydroxy-2-isopropylaminobutyl)-2H-1,4-benzoxazin-3-(4H)-one
- 1-(4-amino-3-chloro-5-trifluoromethylphenyl)-2-tert.-butylamino)ethanol
- 6-hydroxy-8-{1-hydroxy-2-[2-(4-methoxy-phenyl)-1,1-dimethyl-ethylamino]-ethyl}-4H-benzo[1,4]oxazin-3-one
- 6-hydroxy-8-{1-hydroxy-2-[2-(ethyl 4-phenoxy-acetate)-1,1-dimethyl-ethylamino]-ethyl}-4H-benzo[1,4]oxazin-3-one
- 6-hydroxy-8-{1-hydroxy-2-[2-(4-phenoxy-acetic acid)-1,1-dimethyl-ethylamino]-ethyl}-4H-benzo[1,4]oxazin-3-one
- 8-{2-[1,1-dimethyl-2-(2.4.6-trimethylphenyl)-ethylamino]-1-hydroxy-ethyl}-6-hydroxy-4H-benzo[1,4]oxazin-3-one
- 6-hydroxy-8-{1-hydroxy-2-[2-(4-hydroxy-phenyl)-1,1-dimethyl-ethylamino]-ethyl}-4H-benzo[1,4]oxazin-3-one
- 6-hydroxy-8-{1-hydroxy-2-[2-(4-isopropyl-phenyl)-1.1dimethyl-ethylamino]-ethyl}-4H-benzo[1,4]oxazin-3-one
- 8-{2-[2-(4-ethyl-phenyl)-1,1-dimethyl-ethylamino]-1-hydroxy-ethyl}-6-hydroxy-4H-benzo[1,4]oxazin-3-one
- 8-{2-[2-(4-ethoxy-phenyl)-1,1-dimethyl-ethylamino]-1-hydroxy-ethyl}-6-hydroxy-4H-benzo[1,4]oxazin-3-one
- 4-(4-{2-[2-hydroxy-2-(6-hydroxy-3-oxo-3.4-dihydro-2H-benzo[1,4]oxazin-8-yl)-ethylamino]-2-methyl-propyl}-phenoxy)-butyric acid
- 8-{2-[2-(3.4-difluoro-phenyl)-1,1-dimethyl-ethylamino]-1-hydroxy-ethyl}-6-hydroxy-4H-benzo[1,4]oxazin-3-one
- 1-(4-ethoxy-carbonylamino-3-cyano-5-fluorophenyl)-2-(tert-butylamino)ethanol
- 2-hydroxy-5-(1-hydroxy-2-{2-[4-(2-hydroxy-2-phenyl-ethylamino)-phenyl]-ethylamino}-ethyl)-benzaldehyde
- N-[2-hydroxy-5-(1-hydroxy-2-{2 -[4-(2-hydroxy-2-phenyl-ethylamino)-phenyl]-ethylamino}-ethyl)-phenyl]-formamide
- 8-hydroxy-5-(1-hydroxy-2-{2-[4-(6-methoxy-biphenyl-3-ylamino)-phenyl]-ethylamino}-ethyl)-1H-quinolin-2-one
- 8-hydroxy-5-[1-hydroxy-2-(6-phenethylamino-hexylamino)-ethyl]-1H-quinolin-2-one
- 5-[2-(2-{4-[4-(2-amino-2-methyl-propoxy)-phenylamino]-phenyl}-ethylamino)-1-hydroxy-ethyl]-8-hydroxy-1H-quinolin-2-one
- [3-(4-{6-[2-hydroxy-2-(4-hydroxy-3-hydroxymethyl-phenyl)-ethylamino]-hexyloxy}-butyl)-5-methyl-phenyl]-urea
- 4-(2-{6-[2-(2.6-dichloro-benzyloxy)-ethoxy]-hexylamino}-1-hydroxy-ethyl)-2-hydroxymethyl-phenol
- 3-(4-{6-[2-hydroxy-2-(4-hydroxy-3-hydroxymethyl-phenyl)-ethylamino]-hexyloxy}-butyl)-benzylsulphonamide
- 3-(3-{7-[2-hydroxy-2-(4-hydroxy-3-hydroxymethyl-phenyl)-ethylamino]-heptyloxy}-propyl)-benzylsulphonamide
- 4-(2-{6-[4-(3-cyclopentanesulphonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamide
optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

The anticholinergics used are preferably compounds selected from among the tiotropium salts, preferably the bromide salt, oxitropium salts, preferably the bromide salt, flutropium salts, preferably the bromide salt, ipratropium salts, preferably the bromide salt, glycopyrronium salts, preferably the bromide salt, trospium salts, preferably the chloride salt, tolterodine. In the above-mentioned salts the cations are the pharmacologically active constituents. As anions the above-mentioned salts may preferably contain the chloride, bromide, iodide, sulphate, phosphate, methanesulphonate, nitrate, maleate, acetate, citrate, fumarate, tartrate, oxalate, succinate, benzoate or p-toluenesulphonate, while chloride, bromide, iodide, sulphate, methanesulphonate or p-toluenesulphonate are preferred as counter-ions. Of all the salts the chlorides, bromides, iodides and methanesulphonates are particularly preferred.

Other preferred anticholinergics are selected from among the salts of formula **AC.1** 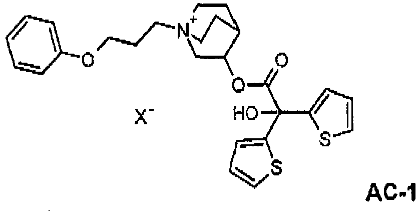 wherein X - denotes an anion with a single negative charge, preferably an anion selected from among the fluoride, chloride, bromide, iodide, sulphate, phosphate, methanesulphonate, nitrate, maleate, acetate, citrate, fumarate, tartrate, oxalate, succinate, benzoate and p-toluenesulphonate, preferably an anion with a single negative charge, particularly preferably an anion selected from among the fluoride, chloride, bromide, methanesulphonate and p-toluenesulphonate, particularly preferably bromide, optionally in the form of the racemates, enantiomers or hydrates thereof. Of particular importance are those pharmaceutical combinations which contain the enantiomers of formula **AC-1-en** 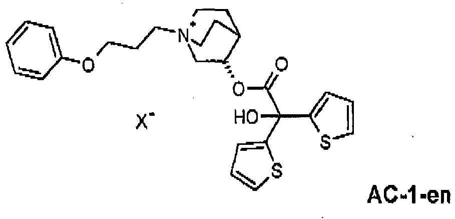 wherein X⁻ may have the above-mentioned meanings. Other preferred anticholinergics are selected from the salts of formula **AC-2** 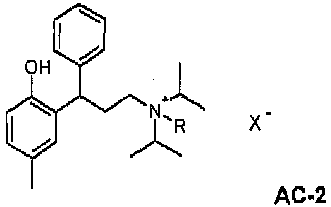 wherein R denotes either methyl or ethyl and wherein X⁻ may have the above-mentioned meanings. In an alternative embodiment the compound of formula **AC-2** may also be present in the form of the free base **AC-2-base**.

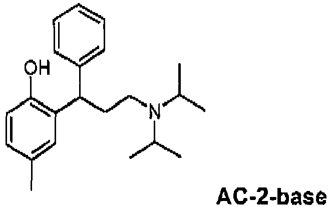

Other specified compounds are:
- tropenol 2,2-diphenylpropionate methobromide,
- scopine 2,2-diphenylpropionate methobromide,
- scopine 2-fluoro-2,2-diphenylacetate methobromide,
- tropenol 2-fluoro-2,2-diphenylacetate methobromide;
- tropenol 3,3',4,4'-tetrafluorobenzilate methobromide,
- scopine 3,3',4,4'-tetrafluorobenzilate methobromide,
- tropenol 4,4'-difluorobenzilate methobromide,
- scopine 4,4'-difluorobenzilate methobromide,
- tropenol 3,3'-difluorobenzilate methobromide,
- scopine 3,3'- difluorobenzilate methobromide;
- tropenol 9-hydroxy-fluorene-9-carboxylate methobromide;
- tropenol 9-fluoro-fluorene-9-carboxylate methobromide;
- scopine 9-hydroxy-fluorene-9- carboxylate methobromide;
- scopine 9-fluoro-fluorene-9- carboxylate methobromide;
- tropenol 9-methyl-fluorene-9- carboxylate methobromide;
- scopine 9-methyl-fluorene-9- carboxylate methobromide;
- cyclopropyltropine benzilate methobromide;
- cyclopropyltropine 2,2-diphenylpropionate methobromide;
- cyclopropyltropine 9-hydroxy-xanthene-9-carboxylate methobromide;
- cyclopropyltropine 9-methyl-fluorene-9-carboxylate methobromide;
- cyclopropyltropine 9-methyl-xanthene-9-carboxylate methobromide;
- cyclopropyltropine 9-hydroxy-fluorene-9-carboxylate methobromide;
- cyclopropyltropine methyl 4,4'-difluorobenzilate methobromide.
- tropenol 9-hydroxy-xanthene-9-carboxylate methobromide;
- scopine 9-hydroxy-xanthene-9-carboxylate methobromide;
- tropenol 9-methyl-xanthene-9-carboxylate -methobromide;
- scopine 9-methyl-xanthene-9-carboxylate -methobromide;
- tropenol 9-ethyl-xanthene-9-carboxylate methobromide;
- tropenol 9-difluoromethyl-xanthene-9-carboxylate methobromide;
- scopine 9-hydroxymethyl-xanthene-9-carboxylate methobromide,

The above-mentioned compounds may also be used as salts within the scope of the present invention, wherein instead of the methobromide the salts metho-X are used, wherein X may have the meanings given hereinbefore for X⁻.

As corticosteroids it is preferable to use compounds selected from among be-clomethasone, betamethasone, budesonide, butixocort, ciclesonide, deflazacort, dexamethasone, etiprednol, flunisolide, fluticasone, loteprednol, mometasone, prednisolone, prednisone, rofleponide, triamcinolone, RPR-106541, NS-126, ST-26 and
- (S)-fluoromethyl 6,9-difluoro-17-[(2-furanylcarbonyl)oxy]-11-hydroxy-16-methyl-3-oxo-androsta-1,4-diene-17-carbothionate
- (S)-(2-oxo-tetrahydro-furan-3S-yl)6,9-difluoro-11-hydroxy-16-methyl-3-oxo-17-propionyloxy-androsta-1,4-diene-17-carbothionate,
- cyanomethyl 6α,9α-difluoro-11β-hydroxy-16α-methyl-3-oxo-17α-(2,2,3,3-tertamethylcyclopropylcarbonyl)oxy-androsta-1,4-diene-17β-carboxylate
optionally in the form of the racemates, enantiomers or diastereomers thereof and optionally in the form of the salts and derivatives thereof, the solvates and/or hydrates thereof. Any reference to steroids includes a reference to any salts or derivatives, hydrates or solvates thereof which may exist. Examples of possible salts and derivatives of the steroids may be: alkali metal salts, such as for example sodium or potassium salts, sulphobenzoates, phosphates, isonicotinates, acetates, dichloroacetates, propionates, dihydrogen phosphates, palmitates, pivalates or furoates.

PDE4-inhibitors which may be used are preferably compounds selected from among enprofyllin, theophyllin, roflumilast, ariflo (cilomilast), tofimilast, pumafentrin, lirimilast, arofyllin, atizoram, D-4418, Bay-198004, BY343, CP-325.366, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370 and
- N-(3,5-dichloro-1-oxo-pyridin-4-yl)-4-difluoromethoxy-3-cyclopropylmethoxybenzamide
- (-)p-[(4aR*, 10*b*S*)-9-ethoxy-1,2,3,4,4a,10b-hexahydro-8-methoxy-2-methylbenzo[s][1,6]naphthyridin-6-yl]-N,N-diisopropylbenzamide
- (R)-(+)-1-(4-bromobenzyl)-4-[(3-cyclopentyloxy)-4-methoxyphenyl]-2-pyrrolidone
- 3-(cyclopentyloxy-4-methoxyphenyl)-1-(4-N'-[N-2-cyano-S-methyl-isothioureido]benzyl)-2-pyrrolidone
- cis[4-cyano-4-(3-cyclopentyloxy-4-methoxyphenyl)cyclohexane-1-carboxylic acid]
- 2-carbamethoxy-4-cyano-4-(3-cyclopropylmethoxy-4-difluoromethoxyphenyl)cyclohexan-1-one
- cis[4-cyano-4-(3-cyclopropylmethoxy-4-difluoromethoxyphenyl)cyclohexan-1-ol]
- (R)-(+)-ethyl[4-(3-cyclopentyloxy-4-methoxyphenyl)pyrrolidin-2-ylidene]acetate
- (S)-(-)-ethyl[4-(3-cyclopentyloxy-4-methoxyphenyl)pyrrolidin-2-ylidene]acetate
- 9-cyclopentyl-5,6-dihydro-7-ethyl-3-(2-thienyl)-9*H*-pyrazolo[3.4-c]-1,2,4-triazolo[4.3-a]pyridine
- 9-cyclopentyl-5,6-dihydro-7-ethyl-3-(*tert*-butyl)-9*H*-pyrazolo[3.4-c]-1,2,4-triazolo[4.3-a]pyridine
optionally in the form of the racemates, enantiomers or diastereomers thereof and optionally in the form of the pharmacologically acceptable acid addition salts thereof, the solvates and/or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrabenzoate and hydro-p-toluenesulphonate.

The LTD4-antagonists used are preferably compounds selected from among montelukast, pranlukast, zafirlukast, MCC-847 (ZD-3523), MN-001, MEN-91507 (LM-1507), VUF-5078, VUF-K-8707, L-733321 and
- 1-(((R)-(3-(2-(6,7-difluoro-2-quinolinyl)ethenyl)phenyl)-3-(2-(2- hydroxy-2-propyl)phenyl)thio)methylcyclopropane-acetic acid,
- 1-(((1(R)-3(3-(2-(2,3-dichlorothieno[3,2-b]pyridin-5-yl)-(E)-ethenyl)phenyl)-3-(2-(1-hydroxy-1-methylethyl)phenyl)propyl)thio)methyl)cyclopropaneacetic acid
- [2-[[2-(4-tert-butyl-2-thiazolyl)-5-benzofuranyl]oxymethyl]phenyl]acetic acid optionally in the form of the racemates, enantiomers or diastereomers thereof and optionally in the form of the pharmacologically acceptable acid addition salts, solvates and/or hydrates thereof. According to the invention the acid addition salts of the betamimetics are preferably selected from among the hydrochloride, hydrobromide, hydroiodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate. By salts or derivatives which the LTD4-antagonists may optionally be capable of forming are meant, for example: alkali metal salts, such as for example sodium or potassium salts, alkaline earth metal salts, sulphobenzoates, phosphates, isonicotinates, acetates, propionates, dihydrogen phosphates, palmitates, pivalates or furoates.

EGFR-inhibitors which may be used are preferably compounds selected from among cetuximab, trastuzumab, ABX-EGF, Mab ICR-62 and
- 4-[(3-chloro-4-fluorophenyl)amino]-6-{[4-(morpholin-4-yl)-1-oxo-2-buten-1-yl]-amino}-7-cyclapropylmethoxy-quinazoline
- 4-[(3-chloro-4-fluorophenyl)amino]-6-{[4-(N,N-diethylamino)-1-oxo-2-buten-1-yl]amino}-7-cyclopropylmethoxy-quinazoline
- 4-[(3-chloro-4-fluorophenyl)amino]-6-{[4-(N,N-dimethylamino)-1-oxo-2-buten-1-yl]amino}-7-cyclopropylmethoxy-quinazoline
- 4-[(R)-(1-phenyl-ethyl)amino]-6-{[4-(morpholin-4-yl)-1-oxo-2-buten-1-yl]-amino}-7-cyclopentyloxy-quinazoline
- 4-[(3-chloro-4-fluoro-phenyl)amino]-6-{[4-((R)-6-methyl-2-oxo-morpholin-4-yl)-1-oxo-2-buten-1-yl]amino}-7-cyclopropylmethoxy-quinazoline
- 4-[(3-chloro-4-fluoro-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-quinazoline
- 4-[(3-chloro-4-fluoro-phenyl)amino]-6-{[4-((R)-2-methoxymethyl-6-oxo-morpholin-4-yl)-1-oxo-2-buten-1-yl]amino}-7-cyclopropylmethoxy-quinazoline
- 4-[(3-chloro-4-fluoro-phenyl)amino]-6-[2-((S)-6-methyl-2-oxo-morpholin-4-yl)-ethoxy]-7-methoxy-quinazoline
- 4-[(3-chloro-4-fluorophenyl)amino]-6-({4-[N-(2-methoxy-ethyl)-N-methyl-amino]-1-oxo-2-buten-1-yl}amino)-7-cyclopropylmethoxy-quinazoline
- 4-[(3-chloro-4-fluorophenyl)amino]-6-{[4-(N,N-dimethylamino)-1-oxo-2-buten-1-yl]amino}-7-cyclopentyloxy-quinazoline
- 4-[(R)-(1-phenyl-ethyl)amino]-6-{[4-(N,N-to-(2-methoxy-ethyl)-amino)-1-oxo-2-buten-1-yl]amino}-7-cyclopropylmethoxy-quinazoline
- 4-[(R)-(1-phenyl-ethyl)amino]-6-({4-[N-(2-methoxy-ethyl)-N-ethyl-amino]-1-oxo-2-buten-1-yl}amino)-7-cyclopropylmethoxy-quinazoline
- 4-[(R)-(1-phenyl-ethyl)amino]-6-({4-[N-(2-methoxy-ethyl)-N-methyl-amino]-1-oxo-2-buten-1-yl}amino)-7-cyclopropylmethoxy-quinazoline
- 4-[(R)-(1-phenyl-ethyl)amino]-6-({4-[N-(tetrahydropyran-4-yl)-N-methyl-amino]-1-oxo-2-buten-1-yl}amino)-7-cyclopropylmethoxy-quinazoline
- 4-[(3-chloro-4-fluorophenyl)amino]-6-{[4-(N,N-dimethylamino)-1-oxo-2-buten-1-yl]amino}-7-((R)-tetrahydrofuran-3-yloxy)-quinazoline
- 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline
- 4-[(3-chloro-4-fluorophenyl)amino]-6-({4-[N-(2-methoxy-ethyl)-N-methyl-amino]-1-oxo-2-buten-1-yl}amino)-7-cyclopentyloxy-quinazoline
- 4-[(3-chloro-4-fluorophenyl)amino]-6-{[4-(N-cyclopropyl-N-methyl-amino)-1-oxo-2-buten-1-yl]amino}-7-cyclopentyloxy-quinazoline
- 4-[(3-chloro-4-fluorophenyl)amino]-6-{[4-(N,N-dimethylamino)-1-oxo-2-buten-1-yl]amino}-7-[(R)-(tetrahydrofuran-2-yl)methoxy]-quinazoline
- 4-[(3-chloro-4-fluorophenyl)amino]-6-{[4-(N,N-dimethylamino)-1-oxo-2-buten-1-yl]amino}-7-[(S)-(tetrahydrofuran-2-yl)methoxy]-quinazoline
- 4-[(3-ethynyl-phenyl)amino]-6.7-to-(2-methoxy-ethoxy)-quinazoline
- 4-[(3-chloro-4-fluorophenyl)amino]-7-[3-(morpholin-4-yl)-propyloxy]-6-[(vinyl-carbonyl)amino]-quinazoline
- 4-[(R)-(1-phenyl-ethyl)amino]-6-(4-hydroxy-phenyl)-7H-pyrrolo[2,3-d]pyrimidine
- 3-cyano-4-[(3-chloro-4-fluorophenyl)amino]-6-{[4-(N,N-dimethylamino)-1-oxo-2-buten-1-yl]amino}-7-ethoxy-quinoline
- 4-{[3-chloro-4-(3-fluoro-benzyloxy)-phenyl]amino}-6-(5-{[(2-methanesulphonyl-ethyl)amino]methyl}-furan-2-yl)quinazoline
- 4-[(R)-(1-phenyl-ethyl)amino]-6-{[4-((R)-6-methyl-2-oxo-morpholin-4-yl)-1-oxo-2-buten-1-yl]amino}-7-methoxy-quinazoline
- 4-[(3-chloro-4-fluorophenyl)amino]-6-{[4-(morpholin-4-yl)-1-oxo-2-buten-1-yl]-amino}-7-[(tetrahydrofuran-2-yl)methoxy]-quinazoline
- 4-[(3-chloro-4-fluorophenyl)amino]-6-({4-[N,N-to-(2-methoxy-ethyl)-amino]-1-oxo-2-buten-1-yl}amino)-7-[(tetrahydrofuran-2-yl)methoxy]-quinazoline
- 4-[(3-ethynyl-phenyl)amino]-6-{[4-(5.5-dimethyl-2-oxo-morpholin-4-yl)-1-oxo-2-buten-1-yl]amino}-quinazoline
- 4-[(3-chloro-4-fluoro-phenyl)amino]-6-[2-(2,2-dimethyl-6-oxo-morpholin-4-yl)-ethoxy]-7-methoxy-quinazoline
- 4-[(3-chloro-4-fluoro-phenyl)amino]-6-[2-(2,2-dimethyl-6-oxo-morpholin-4-yl)-ethoxy]-7-[(R)-(tetrahydrofuran-2-yl)methoxy]-quinazoline
- 4-[(3-chloro-4-fluoro-phenyl)amino]-7-[2-(2,2-dimethyl-6-oxo-morpholin-4-yl)-ethoxy]-6-[(S)-(tetrahydrofuran-2-yl)methoxy]-quinazoline
- 4-[(3-chloro-4-fluoro-phenyl)amino]-6-{2-[4-(2-oxo-morpholin-4-yl)-piperidin-1-yl]-ethoxy}-7-methoxy-quinazoline
- 4-[(3-chloro-4-fluoro-phenyl)amino]-6-[1-(tert.-butyloxycarbonyl)-piperidin-4-yloxy]-7-methoxy-quinazoline
- 4-[(3-chloro-4-fluoro-phenyl)amino]-6-(trans-4-amino-cyclohexan-1-yloxy)-7-methoxy-quinazoline
- 4-[(3-chloro-4-fluoro-phenyl)amino]-6-(trans-4-methanesulphonylamino-cyclohexan-1-yloxy)-7-methoxy-quinazoline
- 4-[(3-chloro-4-fluoro-phenyl)amino]-6-(tetrahydropyran-3-yloxy)-7-methoxy-quinazoline
- 4-[(3-chloro-4-fluoro-phenyl)amino]-6-(1-methyl-piperidin-4-yloxy)-7-methoxy-quinazoline
- 4-[(3-chloro-4-fluoro-phenyl)amino]-6-{1-[(morpholin-4-yl)carbonyl]-piperidin-4-yloxy}-7-methoxy-quinazoline
- 4-[(3-chloro-4-fluoro-phenyl)amino]-6-{1-[(methoxymethyl)carbonyl]-piperidin-4-yloxy}-7-methoxy-quinazoline
- 4-[(3-chloro-4-fluoro-phenyl)amino]-6-(piperidin-3-yloxy)-7-methoxy-quinazoline
- 4-[(3-chloro-4-fluoro-phenyl)amino]-6-[1-(2-acetylamino-ethyl)-piperidin-4-yloxy]-7-methoxy-quinazoline
- 4-[(3-chloro-4-fluoro-phenyl)amino]-6-(tetrahydropyran-4-yloxy)-7-ethoxy-quinazoline
- 4-[(3-chloro-4-fluoro-phenyl)amino]-6-((S)-tetrahydrofuran-3-yloxy)-7-hydroxy-quinazoline
- 4-[(3-chloro-4-fluoro-phenyl)amino]-6-(tetrahydropyran-4-yloxy)-7-(2-methoxy-ethoxy)-quinazoline
- 4-[(3-chloro-4-fluoro-phenyl)amino]-6-{trans-4-[(dimethylamino)sulphonylamino]-cyclohexan-1-yloxy}-7-methoxy-quinazoline
- 4-[(3-chloro-4-fluoro-phenyl)amino]-6-{trans-4-[(morpholin-4-yl)carbonylamino]-cyclohexan-1-yloxy}-7-methoxy-quinazoline
- 4-[(3-chloro-4-fluoro-phenyl)amino]-6-{trans-4-[(morpholin-4-yl)sulphonylamino]-cyclohexan-1-yloxy}-7-methoxy-quinazoline
- 4-[(3-chloro-4-fluoro-phenyl)amino]-6-(tetrahydropyran-4-yloxy)-7-(2-acetylamino-ethoxy)-quinazoline
- 4-[(3-chloro-4-fluoro-phenyl)amino]-6-(tetrahydropyran-4-yloxy)-7-(2-methanesulphonylamino-ethoxy)-quinazoline
- 4-[(3-chloro-4-fluoro-phenyl)amino]-6-{1-[(piperidin-1-yl)carbonyl]-piperidin-4-yloxy}-7-methoxy-quinazoline
- 4-[(3-chloro-4-fluoro-phenyl)amino]-6-(1-aminocarbonylmethyl-piperidin-4-yloxy)-7-methoxy-quinazoline
- 4-[(3-chloro-4-fluoro-phenyl)amino]-6-(cis-4-{N-[(tetrahydropyran-4-yl)carbonyl]-N-methyl-amino}-cyclohexan-1-yloxy)-7-methoxy-quinazoline
- 4-[(3-chloro-4-fluoro-phenyl)amino]-6-(cis-4-{N-[(morpholin-4-yl)carbonyl]-N-methyl-amino}-cyclohexan-1-yloxy)-7-methoxy-quinazoline
- 4-[(3-chloro-4-fluoro-phenyl)amino]-6-(cis-4-{N-[(morpholin-4-yl)sulphonyl]-N-methyl-amino}-cyclohexan-1-yloxy)-7-methoxy-quinazoline
- 4-[(3-chloro-4-fluoro-phenyl)amino]-6-(trans-4-ethanesulphonylamino-cyclohexan-1-yloxy)-7-methoxy-quinazoline
- 4-[(3-chloro-4-fluoro-phenyl)amino]-6-(1-methanesulphonyl-piperidin-4-yloxy)-7-ethoxy-quinazoline
- 4-[(3-chloro-4-fluoro-phenyl)amino]-6-(1-methanesulphonyl-piperidin-4-yloxy)-7-(2-methoxy-ethoxy)-quinazoline
- 4-[(3-chloro-4-fluoro-phenyl)amino]-6-[1-(2-methoxy-acetyl)-piperidin-4-yloxy]-7-(2-methoxy-ethoxy)-quinazoline
- 4-[(3-chloro-4-fluoro-phenyl)amino]-6-(cis-4-acetylamino-cyclohexan-1-yloxy)-7-methoxy-quinazoline
- 4-[(3-ethynyl-phenyl)amino]-6-[1-(tert.-butyloxycarbonyl)-piperidin-4-yloxy]-7-methoxy-quinazoline
- 4-[(3-ethynyl-phenyl)amino]-6-(tetrahydropyran-4-yloxy]-7-methoxy-quinazoline
- 4-[(3-chloro-4-fluoro-phenyl)amino]-6-(cis-4-{N-[(piperidin-1-yl)carbonyl]-N-methyl-amino}-cyclohexan-1-yloxy)-7-methoxy-quinazoline
- 4-[(3-chloro-4-fluoro-phenyl)amino]-6-(cis-4-{N-[(4-methyl-piperazin-1-yl)carbonyl]-N-methyl-amino}-cyclohexan-1-yloxy)-7-methoxy-quinazoline
- 4-[(3-chloro-4-fluoro-phenyl)amino]-6-{cis-4-[(morpholin-4-yl)carbonylamino]-cyclohexan-1-yloxy}-7-methoxy-quinazoline
- 4-[(3-chloro-4-fluoro-phenyl)amino]-6-{1-[2-(2-oxopyrrolidin-1-yl)ethyl]-piperidin-4-yloxy}-7-methoxy-quinazoline
- 4-[(3-chloro-4-fluoro-phenyl)amino]-6-{1-[(morpholin4-yl)carbonyl]-piperidin-4-yloxy}-7-(2-methoxy-ethoxy)-quinazoline
- 4-[(3-ethynyl-phenyl)amino]-6-(1-acetyl-piperidin-4-yloxy)-7-methoxy-quinazoline
- 4-[(3-ethynyl-phenyl)amino]-6-(1-methyl-piperidin-4-yloxy)-7-methoxy-quinazoline
- 4-[(3-ethynyl-phenyl)amino]-6-(1-methanesulphonyl-piperidin-4-yloxy)-7-methoxy-quinazoline
- 4-[(3-chloro-4-fluoro-phenyl)amino]-6-(1-methyl-piperidin-4-yloxy)-7(2-methoxy-ethoxy)-quinazoline
- 4-[(3-chloro-4-fluoro-phenyl)amino]-6-(1-isopropyloxycarbonyl-piperidin-4-yloxy)-7-methoxy-quinazoline
- 4-[(3-chloro-4-fluoro-phenyl)amino]-6-(cis-4-methylamino-cyclohexan-1-yloxy)-7-methoxy-quinazoline
- 4-[(3-chloro-4-fluoro-phenyl)amino]-6-{cis-4-[N-(2-methoxy-acety)-N-methyl-amino]-cyclohexan-1-yloxy}-7-methoxy-quinazoline
- 4-[(3-ethynyl-phenyl)amino]-6-(piperidin-4-yloxy)-7-methoxy-quinazoline
- 4-[(3-ethynyl-phenyl)amino]-6-[1-(2-methoxy-acetyl)-piperidin-4-yloxy]-7-methoxy-quinazoline
- 4-[(3-ethynyl-phenyl)amino]-6-{1-[(morpholin-4-yl)carbonyl]-piperidin-4-yloxy}-7-methoxy-quinazoline
- 4-[(3-chloro-4-fluoro-phenyl)amino]-6-{1-[(cis-2,6-dimethyl-morpholin-4-yl)carbonyl]-piperidin-4-yloxy}-7-methoxy-quinazoline
- 4-[(3-chloro-4-fluoro-phenyl)amino]-6-{1-[(2-methyl-morpholin-4-yl)carbonyl]-piperidin-4-yloxy}-7-methoxy-quinazoline
- 4-[(3-chloro-4-fluoro-phenyl)amino]-6-{1-[(S,S)-(2-oxa-5-aza-bicyclo[2,2,1]hept-5-yl)carbonyl]-piperidin-4-yloxy}-7-methoxy-quinazoline
- 4-[(3-chloro-4-fluoro-phenyl)amino]-6-{1-[(N-methyl-N-2-methoxyethyl-amino)carbonyl]-piperidin-4-yloxy}-7-methoxy-quinazoline
- 4-[(3-chloro-4-fluoro-phenyl)amino]-6-(1-ethyl-piperidin-4-yloxy)-7-methoxy-quinazoline
- 4-[(3-chloro-4-fluoro-phenyl)amino]-6-{1-[(2-methoxyethyl)carbonyl]-piperidin-4-yloxy}-7-methoxy-quinazoline
- 4-[(3-chloro-4-fluoro-phenyl)amino]-6-{1-[(3-methoxypropyl-amino)-carbonyl]-piperidin-4-yloxy}-7-methoxy-quinazoline
- 4-[(3-chloro-4-fluoro-phenyl)amino]-6-[cis-4-(N-methanesulphonyl-N-methyl-amino)-cyclohexan-1-yloxy]-7-methoxy-quinazoline
- 4-[(3-chloro-4-fluoro-phenyl)amino]-6-[cis-4-(N-acetyl-N-methyl-amino)-cyclohexan-1-yloxy]-7-methoxy-quinazoline
- 4-[(3-chloro-4-fluoro-phenyl)amino]-6-(trans-4-methylamino-cyclohexan-1-yloxy)-7-methoxy-quinazoline
- 4-[(3-chloro-4-fluoro-phenyl)amino]-6-[trans-4-(N-methanesulphonyl-N-methyl-amino)-cyclohexan-1-yloxy]-7-methoxy-quinazoline
- 4-[(3-chloro-4-fluoro-phenyl)amino]-6-(trans-4-dimethylamino-cyclohexan-1-yloxy)-7-methoxy-quinazoline
- 4-[(3-chloro-4-fluoro-phenyl)amino]-6-(trans-4-{N-[(morpholin-4-yl)carbonyl]-N-methyl-amino}-cyclohexan-1-yloxy)-7 -methoxy-quinazoline
- 4-[(3-chloro-4-fluora-phenyl)amino]-6-[2-(2,2-dimethyl-6-oxo-morpholin-4-yl)-ethoxy]-7-[(S)-(tetrahydrofuran-2-yl)methoxy]-quinazoline
- 4-[(3-chloro-4-fluoro-phenyl)amino]-6-(1-methanesulphonyl-piperidin-4-yloxy)-7-methoxy-quinazoline
- 4-[(3-chloro-4-fluoro-phenyl)amino]-6-(1-cyano-piperidin-4-yloxy)-7-methoxy-quinazoline
optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

The dopamine agonists used are preferably compounds selected from among bromocriptin, cabergoline, alpha-dihydroergocryptine, lisuride, pergolide, pramipexol, roxindol, ropinirol, talipexol, tergurid and viozan, optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydrooxalate, hydrosuccinate, hydrobenzoate and hydro-p-toluenesulphonate.

H1-Antihistamines which may be used are preferably compounds selected from among epinastine, cetirizine, azelastine, fexofenadine, levocabastine, loratadine, mizolastine, ketotifen, emedastine, dimetindene, clemastine, bamipine, cexchlorpheniramine, pheniramine, doxylamine, chlorophenoxamine, dimenhydrinate, diphenhydramine, promethazine, ebastine, desloratidine and meclozine, optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

It is also possible to use inhalable macromolecules, as disclosed in EP 1 003 478 A1 or CA 2297174 A1.

In addition, the compounds may come from the groups of ergot alkaloid derivatives, the triptans, the CGRP-inhibitors, the phosphodiesterase-V inhibitors, optionally in the form of the racemates, enantiomers or diastereomers thereof, optionally in the form of the pharmacologically acceptable acid addition salts, the solvates and/or hydrates thereof.

Examples of ergot alkaloid derivatives are dihydroergotamine and ergotamine.

### List of reference numbers

- 1: inhaler
- 2: inhalation formulation
- 3: container
- 4: bag
- 5: pressure generator
- 6: holder
- 7: drive spring
- 8: locking element
- 9: conveying tube
- 10: nonreturn valve
- 11: pressure chamber
- 12: expulsion nozzle
- 12a: nozzle protrusion
- 13: mouthpiece
- 14: aerosol
- 15: air supply opening
- 16: upper housing part
- 17: inner part
- 17a: upper part of the inner part
- 17b: lower part of the inner part
- 18: housing part (lower part)
- 19: retaining element
- 20: spring (in the lower housing part)
- 21: container base
- 22: piercing element
- 23: monitoring device
- 24: insert
- 25: hole
- 26: guiding element
- 27: recess
- 28: air flow
- 29: sealing lip

## Claims

1. Inhaler (1) for actively dispensing of an inhalation formulation (2) as an aerosol (14), comprising:
a conveying means (9) for conveying and/or nebulising the inhalation formulation (2), in particular generating the aerosol (14), and
a mouthpiece (13) with at least one fluidically connected air supply opening (15), and
an insert (24) in the mouthpiece (13) in order to define or increase the flow resistance,
**characterized in**
**that** the insert (24) comprises at least one parallel sealing lip (29) to avoid any undesired bypass flow and to achieve a desired clamping within the mouthpiece (13).

2. Inhaler according to claim 1, **characterized in that** the insert (24) can be mounted after assembly of the inhaler (1), in particularly clamped or press-fitted into the mouthpiece (13).

3. Inhaler according to claim 1 or 2, **characterized in that** the inhaler (1) has a flow resistance of at least 60000 Pa^{1/2}s/m³ or at least 75000 Pa^{1/2}s/m³, preferably about 90000 or 96000 Pa^{1/2}s/m³ or more.

4. Inhaler according to one of the previous claims, **characterized in that** the inhaler (1) comprises one or more air supply openings (15).

5. Inhaler according to one of the previous claims, **characterized in that** the inhaler (1) comprises a pressure generator (5) as conveying means, particularly with a conveying element such as a conveying tube (9).

6. Inhaler according to one of the previous claims, **characterized in that** the inhaler (1) comprises a preferably insertable reservoir, such as a container (3), containing the inhalation formulation (2).

7. Inhaler according to one of the previous claims, **characterized in that** the inhaler (1) is designed such that the conveying means (9) generates the aerosol (14) at least after start of the generation independent on any sucking at or breathing in via the mouthpiece (13).

8. Inhaler according to one of the previous claims, **characterized in that** the inhaler (1) is designed such that ambient air is sucked into or flows into the mouthpiece (13) through the air supply opening(s) (15) when the air and the aerosol (14) is sucked via the mouthpiece (13).

9. Inhaler according to one of the previous claims, **characterized in that** the insert (24) is inserted into the mouthpiece (13) sealingly between the inner wall of the mouthpiece (13) and a nozzle protrusion (12a).

10. Inhaler according to one of the previous claims, **characterized in that** the insert (24) is flat or plate-like.

11. Inhaler according to one of the previous claims, **characterized in that** the insert (24) comprises a central recess (27) for a nozzle protrusion (12a) in order to allow preferred mounting between the outer or peripheral surface of the nozzle protrusion (12a) and the inner surface of the mouthpiece (13).

12. Inhaler according to one of the previous claims, **characterized in that** the insert (24) comprises at least one guiding element (26) for deflecting the airflow (28) in order to slow down the flow velocity and/or to generate turbulences.

13. Inhaler according to claim 12, **characterized in that** the guiding element (26) blocks a direct or straight flow of bypass or ambient air through the mouthpiece (13) and/or extends essentially transversally to a main flow direction to outlet direction of the inhaler (1).

14. Inhaler according to claim 12 or 13, **characterized in that** the guiding element (26) is spaced from an associated hole (25) and/or covers an associated hole (25).

15. Inhaler according to one of the previous claims, **characterized in that** the insert (24) comprises at least one hole (25) through which ambient air, which has been sucked in through the at least one opening (15), can flow, wherein the at least one hole (25) is used to define or increase the total flow resistance of the inhaler (1).

## Patentansprüche

1. Inhalator (1) zum aktiven Abgeben einer Inhalationsformulierung (2) als ein Aerosol (14), aufweisend:
ein Fördermittel (9) zum Fördern und/oder Zerstäuben der Inhalationsformulierung (2), insbesondere Erzeugen des Aerosols (14), und
ein Mundstück (13) mit mindestens einer fluidisch verbundenen Luftversorgungsöffnung (15), und
einen Einsatz (24) in dem Mundstück (13), um den Strömungswiderstand zu definieren oder erhöhen,
**dadurch gekennzeichnet,**
**dass** der Einsatz (24) mindestens eine parallele Dichtlippe (29) aufweist, um eine unerwünschte Nebenströmung zu vermeiden und um ein gewünschtes Klemmen innerhalb des Mundstücks (13) zu erreichen.

2. Inhalator gemäß Anspruch 1, **dadurch gekennzeichnet, dass** der Einsatz (24) nach Zusammenbau des Inhalators (1) angebracht, insbesondere in das Mundstück (13) geklemmt oder gepresst, werden kann.

3. Inhalator gemäß Anspruch 1 oder 2, **dadurch gekennzeichnet, dass** der Inhalator (1) einen Strömungswiderstand von mindestens 60000 Pa^{1/2}s/m³ oder mindestens 75000 Pa^{1/2} s/m³, vorzugsweise etwa 90000 oder 96000 Pa^{1/2}s/m³ oder mehr hat.

4. Inhalator gemäß einem der vorherigen Ansprüche, **dadurch gekennzeichnet, dass** der Inhalator (1) eine oder mehr Luftversorgungsöffnungen (15) aufweist.

5. Inhalator gemäß einem der vorherigen Ansprüche, **dadurch gekennzeichnet, dass** der Inhalator (1) einen Druckerzeuger (5) als Fördermittel aufweist, insbesondere mit einem Förderelement, wie einem Förderrohr (9).

6. Inhalator gemäß einem der vorherigen Ansprüche, **dadurch gekennzeichnet, dass** der Inhalator (1) ein vorzugsweise einsetzbares Reservoir, wie einen Behälter (3), enthaltend die Inhalationsformulierung (2), aufweist.

7. Inhalator gemäß einem der vorherigen Ansprüche, **dadurch gekennzeichnet, dass** der Inhalator (1) derart ausgebildet ist, dass das Fördermittel (9) das Aerosol (14) zumindest nach Beginn der Erzeugung unabhängig von einem Saugen oder Atmen durch das Mundstück (13) erzeugt.

8. Inhalator gemäß einem der vorherigen Ansprüche, **dadurch gekennzeichnet, dass** der Inhalator (1) derart ausgebildet ist, dass Umgebungsluft durch die Luftversorgungsöffnung(en) (15) in das Mundstück (13) gesaugt wird oder strömt, wenn die Luft und das Aerosol (14) über das Mundstück (13) gesaugt werden.

9. Inhalator gemäß einem der vorherigen Ansprüche, **dadurch gekennzeichnet, dass** der Einsatz (24) dichtend zwischen der inneren Wandung des Mundstücks (13) und einem Düsenvorsprung (12a) in das Mundstück (13) eingesetzt ist.

10. Inhalator gemäß einem der vorherigen Ansprüche, **dadurch gekennzeichnet, dass** der Einsatz (24) flach oder plattenartig ist.

11. Inhalator gemäß einem der vorherigen Ansprüche, **dadurch gekennzeichnet, dass** der Einsatz (24) eine zentrale Aussparung (27) für einen Düsenvorsprung (12a) aufweist, um eine bevorzugte Befestigung zwischen der äußeren oder peripheren Fläche des Düsenvorsprungs (12a) und der inneren Fläche des Mundstücks (13) zu gestatten.

12. Inhalator gemäß einem der vorherigen Ansprüche, **dadurch gekennzeichnet, dass** der Einsatz (24) mindestens ein Führungselement (26) zum Ablenken des Luftstroms (28) aufweist, um die Strömungsgeschwindigkeit zu verringern und/oder Turbulenzen zu erzeugen.

13. Inhalator gemäß Anspruch 12, **dadurch gekennzeichnet, dass** das Führungselement (26) einen direkten oder geraden Strom von Neben- oder Umgebungsluft durch das Mundstück (13) blockiert und/oder sich im Wesentlichen transversal zu einer Hauptströmungsrichtung zur Auslassrichtung des Inhalators (1) erstreckt.

14. Inhalator gemäß Anspruch 12 oder 13, **dadurch gekennzeichnet, dass** das Führungselement (26) von einem zugeordneten Loch (25) beabstandet ist und/oder ein zugeordnetes Loch (25) bedeckt.

15. Inhalator gemäß einem der vorherigen Ansprüche, **dadurch gekennzeichnet, dass** der Einsatz (24) mindestens ein Loch (25) aufweist, durch welches Umgebungsluft, die durch die mindestens eine Öffnung (15) eingesaugt wurde, strömen kann, wobei das mindestens eine Loch (25) dazu verwendet wird, den Gesamtströmungswiderstand des Inhalators (1) zu definieren oder erhöhen.

## Revendications

1. Inhalateur (1) pour distribuer activement une formule d'inhalation (2), en tant qu'aérosol (14) comprenant:
un moyen de transport (9) pour transporter et/ou nébuliser la formule d'inhalation (2), en particulier générer l'aérosol (14), et
un embout buccal (13) avec au moins une ouverture d'alimentation en air (15) raccordée de manière fluidique, et
un insert (24) dans l'embout buccal (13) afin de définir ou augmenter la résistance à l'écoulement,
**caractérisé**
**en ce que** l'insert (24) comprend au moins une lèvre d'étanchéité parallèle (29) pour éviter tout écoulement de dérivation indésiré et pour obtenir un serrage souhaité à l'intérieur de l'embout buccal (13).

2. Inhalateur selon la revendication 1, **caractérisé en ce que** l'insert (24) peut être monté après l'assemblage de l'inhalateur (1) en particulier serré ou monté à la presse dans l'embout buccal (13).

3. Inhalateur selon la revendication 1 ou 2, **caractérisé en ce que** l'inhalateur (1) a une résistance à l'écoulement d'au moins 60 000 Pa1/2 s/m3 ou au moins 75 000 Pa1/2 s/m3, de préférence d'environ 90 000 ou 96 000 Pa1/2 s/m3 ou plus.

4. Inhalateur selon l'une des revendications précédentes, **caractérisé en ce que** l'inhalateur (1) comprend une ou plusieurs ouvertures d'alimentation en air (15).

5. Inhalateur selon l'une des revendications précédentes, **caractérisé en ce que** l'inhalateur (1) comprend un générateur de pression (5) en tant que moyen de transport, en particulier avec un élément de transport tel qu'un tube de transport (9).

6. Inhalateur selon l'une des revendications précédentes, **caractérisé en ce que** l'inhalateur (1) comprend un réservoir de préférence insérable, tel qu'un récipient (3), contenant la formule d'inhalation (2).

7. Inhalateur selon l'une des revendications précédentes, **caractérisé en ce que** l'inhalateur (1) est conçu de sorte que le moyen de transport (9) génère l'aérosol (14) au moins après le début de la génération indépendamment de toute aspiration ou de respiration via l'embout buccal (13).

8. Inhalateur selon l'une des revendications précédentes, **caractérisé en ce que** l'inhalateur (1) est conçu de sorte que l'air ambiant est aspiré dans ou s'écoule dans l'embout buccal (13) par l'ouverture (les ouvertures) d'alimentation en air (15) lorsque l'air et l'aérosol (14) sont aspirés via l'embout buccal (13).

9. Inhalateur selon l'une des revendications précédentes, **caractérisé en ce que** l'insert (24) est inséré dans l'embout buccal (13) de manière étanche entre la paroi interne de l'embout buccal (13) et la saillie de buse (12a).

10. Inhalateur selon l'une des revendications précédentes, **caractérisé en ce que** l'insert (24) est plat ou en forme de plaque.

11. Inhalateur selon l'une des revendications précédentes, **caractérisé en ce que** l'insert (24) comprend un évidement central (27) pour une saillie de buse (12a) afin de permettre le montage préféré entre la surface externe ou périphérique de la saillie de buse (12a) et la surface interne de l'embout buccal (13).

12. Inhalateur selon l'une des revendications précédentes, **caractérisé en ce que** l'insert (24) comprend au moins un élément de guidage (26) pour dévier l'écoulement d'air (28) afin de ralentir la vitesse d'écoulement et/ou de générer des turbulences.

13. Inhalateur selon la revendication 12, **caractérisé en ce que** l'élément de guidage (26) bloque un écoulement direct ou droit de dérivation ou d'air ambiant à travers l'embout buccal (13) et/ou s'étend essentiellement transversalement par rapport à la direction d'écoulement principale vers la direction de sortie de l'inhalateur (1).

14. Inhalateur selon la revendication 12 ou 13, **caractérisé en ce que** l'élément de guidage (26) est espacé d'un trou associé (25) et/ou recouvre un trou associé (25).

15. Inhalateur selon l'une des revendications précédentes, **caractérisé en ce que** l'insert (24) comprend au moins un trou (25) à travers lequel l'air ambiant, qui a été aspiré par la au moins une ouverture (15), peut s'écouler, dans lequel le au moins un trou (25) est utilisé pour définir ou augmenter la résistance totale à l'écoulement de l'inhalateur (1).
